(19) Europäisches Patentamt
European Patent Office
Office européen des brevets

(11) Veröffentlichungsnummer: **0 323 647 B1**

(12) # EUROPÄISCHE PATENTSCHRIFT

(45) Veröffentlichungstag der Patentschrift: **30.10.91 Patentblatt 91/44**

(51) Int. Cl.[5]: **C07D 233/32,** C07D 239/10

(21) Anmeldenummer: **88121874.7**

(22) Anmeldetag: **30.12.88**

(54) **Verfahren zur Herstellung von cyclischen N,N'-Dimethylharnstoffen.**

(30) Priorität: **05.01.88 DE 3800083**

(43) Veröffentlichungstag der Anmeldung: **12.07.89 Patentblatt 89/28**

(45) Bekanntmachung des Hinweises auf die Patenterteilung: **30.10.91 Patentblatt 91/44**

(84) Benannte Vertragsstaaten: **AT BE DE ES FR GB NL SE**

(56) Entgegenhaltungen:
**GB-A- 1 517 820**
**US-A- 4 617 400**
**US-A- 4 642 351**

(73) Patentinhaber: **BASF Aktiengesellschaft**
**Carl-Bosch-Strasse 38**
**W-6700 Ludwigshafen (DE)**

(72) Erfinder: **Franz, Lothar, Dr.**
**Sternstrasse 197**
**W-6700 Ludwigshafen (DE)**
Erfinder: **Eggersdorfer, Manfred, Dr.**
**Hannongstrasse 18**
**W-6710 Frankenthal (DE)**
Erfinder: **Voges, Dieter, Dr.**
**Richard-Wagner-Strasse 28**
**W-6800 Mannheim 1 (DE)**

**EP 0 323 647 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft die Herstellung von cyclischen N,N-Dimethylharnstoffen durch katalytische Hydrierung von cyclischen Harnstoffen, die in α-Stellung zu den beiden Stickstoffatomen Hydroxygruppen tragen.

Es ist bekannt, z.B. aus der britischen Patentschrift 1517820 oder der US-Patentschrift 4617400, cyclische N,N'-Dimethylharnstoffe durch katalytische Hydrierung der entsprechenden N,N'-Dimethylolharnstoffe unter Verwendung von Palladium-Aktivkohle-Hydrierkatalysatoren herzustellen.

Die bekannten Verfahren sind jedoch unbefriedigend, da für die Hydrierung ein hoher Edelmetalleinsatz, bezogen auf die zu hydrierenden N,N'-Dimethylolharnstoffe, erforderlich ist. Hinzu kommt, daß wegen der Verwendung von Aktivkohle als Trägermaterial eine Regenerierung der verbrauchten Katalysatoren mit den üblicherweise verwendeten Methoden, z.B. durch Abbrennen von Ablagerungen, nicht möglich ist.

Es bestand daher Bedarf nach einem Verfahren zur Herstellung von cyclischen N,N'-Dimethylharnstoffen, bei dem die Nachteile der bekannten Verfahren vermieden werden können.

Es wurde nun ein vorteilhaftes Verfahren gefunden zur Herstellung von cyclischen N,N'-Dimethylharnstoffen der allgemeinen Formel (I)

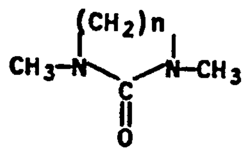

(I),

in der n für 2 oder 3 steht, durch Hydrierung von cyclischen Harnstoffen der allgemeinen Formel (II)

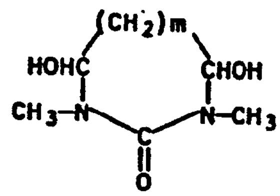

(II),

in der m für 0 oder 1 steht, oder der allgemeinen Formel (III)

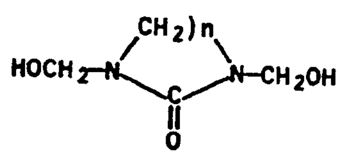

(III),

in der n für 2 oder 3 steht, in Gegenwart von Wasserstoff und einem Hydrierkatalysator, welches dadurch gekennzeichnet ist, daß als Hydrierkatalysator Palladium auf einem anorganischen Trägermaterial verwendet wird und der Trägerkatalysator zusätzlich Schwefel, Selen und/oder Tellur enthält.

Nach dem neuen Verfahren können auch bei Verwendung von relativ geringen Mengen Hydrierkatalysator und dementsprechend geringem Einsatz der Palladiumkomponente, bezogen auf die zu hydrierenden cyclischen Dihydroxyharnstoffe, gute Ausbeuten an den cyclischen N,N'-Dimethylharnstoffen erzielt werden. Außerdem läßt sich der Katalysator in einfacher Weise, z.B. durch Abbrennen, regenerieren.

Als Ausgangsstoffe für das erfindungsgemäße Verfahren werden cyclische Harnstoffe der allgemeinen Formel (II)

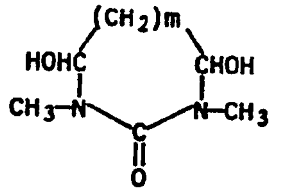

(II),

in der m für 0 oder 1 steht (vgl. Liebigs Ann.Chem. 726, 89-99 (1969)), verwendet. Als weitere Ausgangsstoffe werden cyclische Harnstoffe der allgemeinen Formel (III)

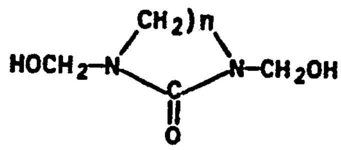

(III),

in der n für 2 oder 3 steht, z.B. N,N'-Dimethylolethylenharnstoff oder N,N-Dimethylolpropylenharnstoff, eingesetzt.

Die cyclischen Harnstoffe der allgemeinen Formel (III) können als solche eingesetzt werden. Es ist jedoch auch möglich, die cyclischen Harnstoffe der allgemeinen Formel (III) zunächst in einem Reaktionsgefäß durch Umsetzung von cyclischen Harnstoffen der allgemeinen Formel (IV)

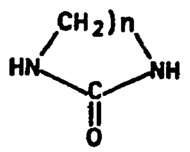

(IV),

in der n für 2 oder 3 steht, mit Formaldehyd in Gegenwart von Wasserstoff und dem erfindungsgemäßen Hydrierkatalysator herzustellen und die entstehenden cyclischen N,N'-Dimethylolharnstoffe der allgemeinen Formel (III) in dem Maße, wie sie entstehen, zu den cyclischen N,N'-Dimethylharnstoffen der allgemeinen Formel (I) zu hydrieren. Bei dieser Arbeitsweise gelten die für die Hydrierung angewendeten Reaktionsbedingungen wie Druck und Temperatur gleichzeitig für die vorhergehende Umsetzung mit Formaldehyd zu den als Zwischenprodukt entstehenden cyclischen N,N'-Dimethylolharnstoffen.

Für die Herstellung der cyclischen Harnstoffe der allgemeinen Formel (III) kann Formaldehyd als solcher eingesetzt werden, z.B. in wäßriger oder methanolischer Lösung. Es können jedoch auch unter den Reaktionsbedingungen Formaldehyd abgebende Stoffe wie Paraformaldehyd oder Trioxan verwendet werden. Der Formaldehyd wird im allgemeinen in der stöchiometrischen Menge oder im Überschuß, z.B. in Mengen bis zur doppelten stöchiometrischen Menge, eingesetzt.

Als Hydrierkatalysator wird erfindungsgemäß Palladium auf einem anorganischen Trägermaterial verwendet, wobei der Trägerkatalysator zusätzlich Schwefel, Selen und/oder Tellur enthält. Als anorganische Trägermaterialien werden im allgemeinen Carbonate, Oxide, Phosphate, Silikate und Sulfate der Elemente der II., III. und IV.-Hauptgruppe des Periodensystems verwendet. Geeignete Trägermaterialien sind beispielsweise Aluminiumoxid, Kieselgel, Titandioxid, Magnesiumoxid. Vorzugsweise wird Aluminiumoxid, insbesondere γ-Aluminiumoxid, verwendet. Der Palladiumgehalt der Hydrierkatalysatoren beträgt im allgemeinen 0,1 bis 4 Gew.%, vorzugsweise 0,5 bis 4 Gew.%, insbesondere 0,5 bis 2 Gew.%, bezogen auf den gesamten Trägerkatalysator. Der Gehalt an den Chalkogenen Schwefel, Selen und/oder Tellur beträgt zweckmäßig 0,1 bis 5 Gew.%, vorzugsweise 0,2 bis 3 Gew.%, insbesondere 0,2 bis 1,5 Gew.%, bezogen auf den gesamten Trägerkatalysator.

Es kann vorteilhaft sein, den Hydrierkatalysator zusätzlich mit Seltenerdmetallen wie Scandium, Yttrium, Cer, zu dotieren. Im allgemeinen beträgt der Gehalt der Hydrierkatalysatoren an den Seltenerdmetallen, die im allgemeinen in Form der Oxide eingesetzt werden, 0,05 bis 10 Gew.%, vorzugsweise 0,05 bis 5 Gew.%, insbesondere 0,1 bis 5 Gew.%, gerechnet als Oxide und bezogen auf den Trägerkatalysator. Der Hydrierkatalysator wird zweckmäßig in solchen Mengen eingesetzt, daß das Gewichtsverhältnis des im Hydrierkatalysator enthaltenen Palladiums zu den zu hydrierenden cyclischen Dihydroxyharnstoffen 1 : 10.000 bis 50 : 1.000, vorzugsweise 1 : 10.000 bis 15 : 1.000, insbesondere 5 : 10.000 bis 10 : 1.000 beträgt.

Die Hydrierung wird zweckmäßig in einem Lösungsmittel durchgeführt. Geeignete Lösungsmittel sind beispielsweise Wasser und polare organische Lösungsmittel, z.B. Alkohole wie Methanol, Ethanol, Propanol, Isopropanol, Butanol, Isobutanol.

Es kann zweckmäßig sein, die Hydrierung in Gegenwart von Mineralsäuren wie Schwefelsäure oder Phosphorsäure, organischen Sulfonsäuren oder von Ionenaustauschern durchzuführen. Geeignete organische Sulfonsäuren sind z.B. Benzolsulfonsäure, p-Toluolsulfonsäure. Als Ionenaustauscher kommen vorzugsweise Ionenaustauscher in der Wasserstofform in Betracht. Geeignete Ionenaustauscher sind beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie kernsulfonierte vernetzte Styrol-Divinylbenzol-Copolymerisate. Die Mineralsäuren, organischen Sulfonsäuren und Ionenaustauscher werden im allgemeinen in Mengen von 0,5 bis 100 Gew.%, vorzugsweise 0,5 bis 50 Gew.%, insbesondere 1 bis 20 Gew.%, bezogen auf die zu hydrierenden cyclischen Dihydroxyharnstoffe, verwendet.

Die Hydrierung wird im allgemeinen bei Temperaturen von 80 bis 200°C, vorzugsweise von 100 bis 180°C, insbesondere 110 bis 160°C, und zweckmäßig bei erhöhtem Druck, im allgemeinen bei einem Druck von 20 bis 300 bar, vorzugsweise 30 bis 200 bar, insbesondere 40 bis 150 bar, durchgeführt.

Die Hydrierung kann diskontinuierlich, z.B. in einem Autoklaven, oder kontinuierlich durchgeführt werden.

Die erfindungemäß erhältlichen cyclischen N,N'-Dimethylharnstoffe finden beispielsweise als Lösungsmittel Verwendung.

Die folgenden Beispiele veranschaulichen die Erfindung.

Beispiel 1

In einen 3,5 1-Rührautoklaven werden 1500 g einer 50 gew.%igen wäßrigen Lösung von N,N'-Dimethylolpropylenharnstoff, 100 g 85 gew.%ige Phosphorsäure und 10 g Hydrierkatalysator (1,15 Gew.% PdO, 0,25 Gew.% $TeO_2$, jeweils bezogen auf den Gesamthydrierkatalysator, auf $\gamma$-$Al_2O_3$) gegeben. Bei einer Temperatur von 120°C und einem Druck von 80 bar, der durch Aufpressen des Hydrierwasserstoffs erzeugt wird, wird die Reaktionsmischung 10 h hydriert. Man trennt den Katalysator ab. Bei der Destillation im Vakuum erhält man 375,4 g (62,4%) N,N'-Dimethylpropylenharnstoff.

Beispiel 2

In einen 3,5 1-Rührautoklaven gibt man 1500 g einer 50 gew.%igen wäßrigen Lösung von N,N'-Dimethylolpropylenharnstoff, 100 g 85 gew.%ige Phosphorsäure und 10 g Hydrierkatalysator (1% Pd, 0,2 Gew.% Se, jeweils bezogen auf den Katalysator, auf $\gamma$-$Al_2O_3$). Bei einer Temperatur von 120°C und einem Druck von 80 bar, der durch Aufpressen des Hydrierwasserstoffs erzeugt wird, wird die Reaktionsmischung 10 h hydriert. Man trennt den Katalysator ab und erhält bei der anschließenden Destillation im Vakuum 421,9 g (70,3%) N,N'-Dimethylpropylenharnstoff.

Beispiel 3

In einen 3,5 1-Rührautoklaven gibt man 1500 g einer 50 gew.%igen wäßrigen Lösung von N,N'-Dimethylolpropylenharnstoff, 100 g 85 gew.%ige Phosphorsäure und 10 g Katalysator (1,15% PdO, 0,5% $CeO_2$, 0,28% $SeO_2$, jeweils bezogen auf den Katalysator, auf $\gamma$-$Al_2O_3$). Bei einer Temperatur von 120°C und einem Druck von 80 bar, der durch Aufpressen des Hydrierwasserstoffs erzeugt wird, wird die Reaktionsmischung 10 h hydriert. Man trennt den Katalysator ab und erhält bei der anschließenden Destillation im Vakuum 350 g (58%) N,N'-Dimethylpropylenharnstoff.

Beispiel 4

In einen 3,5 1-Rührautoklaven gibt man 1500 g einer 50 gew.%igen wäßrigen Lösung von N,N'-Dimethylolpropylenharnstoff, 100 g 85 gew.%ige Phosphorsäure und 10 g Katalysator (1% Pd, 1% S, jeweils bezogen auf den Katalysator, auf $\gamma$-$Al_2O_3$). Bei einer Temperatur von 120°C und einem Druck von 80 bar, der durch Aufpressen des Hydrierwasserstoffs erzeugt wird, wird die Reaktionsmischung 5 h hydriert. Man trennt den Katalysator ab und destilliert das Filtrat im Vakuum. Dabei erhält man 537,5 g (90%) N,N'-Dimethylpropylenharnstoff.

Beispiel 5

Herstellung von Katalysatoren gemäß der Erfindung

a) Zu 2870 Gew.-Teilen gamma-Aluminiumoxid gibt man ein Gemisch aus 250 Gew.-Teilen einer 11 gew.-%igen Palladiumnitratlösung, 1250 Gew.-Teilen dest. Wasser, 8,95 Gew.-Teilen selenige Säure und 140 Gew.-Teilen Salpetersäure. In einer Trockentrommel wird das Wasser bei 120°C abgedampft und der Rückstand bei 520°C 6 h calciniert.

b) Zu 495 Gew.-Teilen gamma-Aluminiumoxid gibt man ein Gemisch aus 45 Gew.-Teilen einer 11 gew.-%igen Palladiumnitratlösung, 1,82 Gew.-Teilen Tellursäure und 606 Gew.-Teilen Wasser. Trocknung und Calcinierung führt man wie unter a) durch.

**Patentansprüche**

1. Verfahren zur Herstellung von cyclischen N,N'-Dimethylharnstoffen der allgemeinen Formel (I)

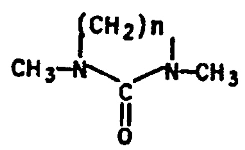

(I),

in der n für 2 oder 3 steht, durch Hydrierung von cyclischen Harnstoffen der allgemeinen Formel (II)

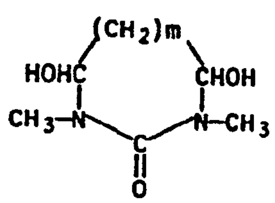

(II),

in der m für 0 oder 1 steht, oder der allgemeinen Formel (III)

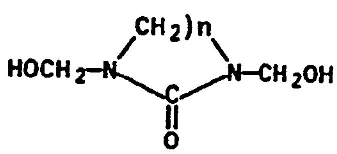

(III),

in der n für 2 oder 3 steht, in Gegenwart von Wasserstoff und einem Hydrierkatalysator, dadurch gekennzeichnet, daß als Hydrierkatalysator Palladium auf einem anorganischen Trägermaterial verwendet wird und der Trägerkatalysator zusätzlich Schwefel, Selen und/oder Tellur enthält.

2. Verfahren nach Anspruch 1, dadurch gekennzeichnet, daß die cyclischen Harnstoffe der allgemeinen Formel (III) durch Umsetzung von cyclischen Harnstoffen der allgemeinen Formel (IV)

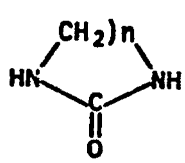

(IV),

in der n für 2 oder 3 steht, mit Formaldehyd in Gegenwart von Wasserstoff und dem Hydrierkatalysator hergestellt werden und die entstehenden cyclischen Harnstoffe der allgemeinen Formel (III) sogleich zu den cyclischen N,N-Dimethylharnstoffen der allgemeinen Formel (I) hydriert werden.

3. Verfahren nach Anspruch 1 oder 2, dadurch gekennzeichnet, daß die Hydrierung in Gegenwart von Mineralsäuren, organischen Sulfonsäuren oder Ionenaustauschern durchgeführt wird.

4. Verfahren nach Anspruch 1 bis 3, dadurch gekennzeichnet, daß der Katalysator zusätzlich mit Seltenerdmetallen dotiert ist.

## Claims

1. A process for the preparation of a cyclic N,N'-dimethylurea of the formula (I)

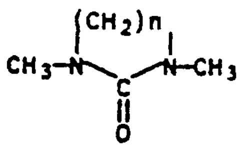

(I)

where n is 2 or 3, by hydrogenating a cyclic urea of the formula (II)

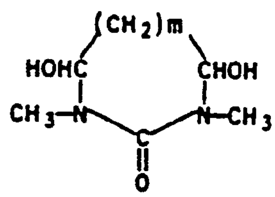

(II)

where m is 0 or 1, or of the formula (III)

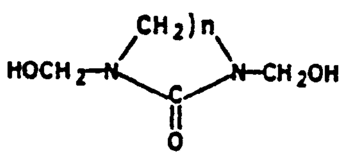

(III)

where n is 2 or 3, in the presence of hydrogen and of a hydrogenation catalyst, wherein the hydrogenation catalyst used is palladium on an inorganic carrier, and the supported catalyst additionally contains sulfur, selenium and/or tellurium.

2. A process as claimed in claim 1, wherein the cyclic urea of the formula (III) is prepared by reacting a cyclic urea of the formula (IV)

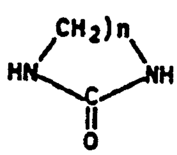

(IV)

where n is 2 or 3, with formaldehyde in the presence of hydrogen and of the hydrogenation catalyst, and the corresponding cyclic urea of the formula (III) is immediately hydrogenated to give a cyclic N,N'-dimethylurea of the formula (I).

3. A process as claimed in claim 1 or 2, wherein the hydrogenation is carried out in the presence of a mineral acid, an organic sulfonic acid or an ion exchanger.

4. A process as claimed in any of claims 1 to 3, wherein the catalyst is additionally doped with a rare earth metal.

**Revendications**

1. Procédé de préparation de N,N'-diméthylurées cycliques de formule générale (I)

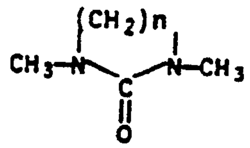

(I),

dans laquelle n est mis pour 2 ou 3, par hydrogénation d'urées cycliques de formule générale (II)

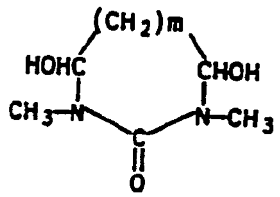

(II),

dans laquelle m est mis pour 0 ou 1, ou de formule générale (III)

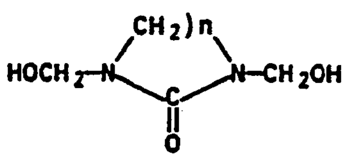

(III),

dans laquelle n est mis pour 2 ou 3, en présence d'hydrogène et d'un catalyseur d'hydrogénation, caractérisé en ce qu'on utilise, comme catalyseur l'hydrogénation, du palladium fixé sur une matière de support inorganique, et en ce que le catalyseur fixé sur support contient en plus du soufre, du sélénium et/ou du tellure.

2. Procédé selon la revendication 1, caractérisé en ce que les urées cycliques de formule générale (III) sont préparées par réaction d'urées cycliques de formule générale (IV)

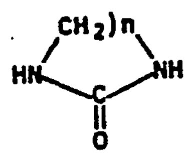

(IV),

dans laquelle n est mis pour 2 ou 3, avec du formaldéhyde en présence d'hydrogène et du catalyseur d'hydrogénation, et en ce que les urées cycliques de formule générale (III) formées sont immédiatement hydrogénées pour donner les N,N'-diméthylurées cycliques de formule générale (I).

3. Procédé selon la revendication 1 ou 2, caractérisé en ce que l'hydrogénation est conduite en présence d'acides minéraux, d'acides sulfoniques organiques ou d'échangeurs d'ions.

4. Procédé selon l'une quelconque des revendications 1 à 3, caractérisé en ce que le catalyseur est dopé en plus avec des métaux des terres rares.